# FASCICULE DE BREVET EUROPEEN

(11) **EP 0 705 597 B1**
(45) Date de publication et mention de la délivrance du brevet: **24.09.1997**
(21) Numéro de dépôt: 95401907.1
(22) Date de dépôt: 17.08.1995
(51) Int. Cl.: A61K 7/13, A45D 7/00, A45D 19/00

(54) **Procédé de coloration directe des fibres kératiniques à l'aide de colorants directs cationiques et de vapeur d'eau**
Verfahren zum direkten Färben von Keratinfasern mit kationischen Direktfarbstoffen und Wasserdampf
Process of direct hair dyeing using cationic direct dyes and steam

(30) Priorité: 21.09.1994 FR 9411265
(43) Date de publication de la demande: 10.04.1996
(73) Titulaire: L'OREAL, 75008 Paris (FR)
(72) Inventeur: Audousset, Marie-Pascale, F-92600 Asnieres (FR)
(74) Mandataire: Tezier Herman, Béatrice

(56) Documents cités:
- EP-A- 0 103 547
- CH-A- 357 161
- DE-A- 4 235 436
- DE-U- 9 318 614
- FR-A- 1 011 151
- FR-A- 1 157 665
- FR-A- 2 273 492
- GB-A- 2 168 082

## Description

La présente invention est relative à un procédé de coloration (ou teinture) directe des fibres kératiniques mettant en oeuvre de la vapeur d'eau et une composition tinctoriale comprenant au moins un colorant direct cationique.

L'emploi de colorants cationiques pour la teinture directe des fibres kératiniques et en particulier des fibres kératiniques humaines telles que les cheveux, est bien connu dans l'état de la technique. Ces colorants directs, du fait de leur caractère cationique présentent une bonne affinité pour les fibres. Ces colorants cationiques composent une gamme de couleurs assez variée permettant une formulation dans toutes les tonalités.

Cependant, ces colorants sont très sensibles au degré de sensibilisation (i.e l'état de dégradation) de la fibre : ils sont sélectifs.

On appelle sélectivité d'un colorant la différence de montée (i.e de pouvoir de coloration) de celui-ci sur les fibres capillaires ou leurs différentes parties selon que celles-ci ont été plus ou moins sensibilisées (i.e "abîmées") soit par un traitement tel qu'une décoloration ou une permanente, soit par les agents atmosphériques.

Les résultats de coloration obtenus sur des cheveux présentant des différences de sensibilisation sont donc hétérogènes. Ces irrégularités ne sont bien évidemment pas souhaitables d'un point de vue esthétique.

La présente invention vise à résoudre le problème ci-dessus.

La demanderesse a maintenant découvert de façon surprenante que l'utilisation d'un gaz chauffé à une température supérieure à 75°C comprenant de la vapeur d'eau sur des cheveux traités avec au moins un colorant direct cationique sélectionné permettait d'obtenir des résultats tinctoriaux dépendant peu du degré de sensibilisation des fibres kératiniques à colorer.

Selon l'invention, les cheveux sont ainsi colorés de façon uniforme des racines aux pointes sur l'ensemble de la chevelure ou sur certaines mèches sélectionnées et ceci quel que soit l'état du cheveu.

La coloration est très rapide et les cheveux présentent d'excellentes propriétés cosmétiques.

On notera que l'utilisation de la vapeur d'eau dans un procédé de coloration d'oxydation a déjà été décrite dans le brevet FR1011151, document dans lequel de la vapeur d'eau chauffée à environ 50°C est mise en oeuvre dans le but d'accélérer le processus de coloration d'oxydation des cheveux tout en diminuant les doses de teintures employées. Toutefois, à cette température, il n'y a pas de diminution de la sélectivité au sens défini ci-avant pour des colorants cationiques. L'utilisation de la vapeur d'eau a également été décrite dans un procédé de teinture capillaire au vert de méthylène, qui est une thiazine cationique, dans le document FR-A-2 273 492, dans lequel une injection de vapeur d'eau surchauffée à 105°C est mise en oeuvre pendant 30 secondes pour obtenir un pouvoir tinctorial élevé.

La présente invention concerne ainsi un procédé de teinture directe des fibres kératiniques mettant en oeuvre au moins un colorant direct cationique et un gaz contenant de la vapeur d'eau, caractérisé par le fait que ledit colorant direct cationique est choisi parmi les naphtoquinones et anthraquinones cationiques, les méthines cationiques, les azoïques cationiques, les oxazines cationiques et les sels desdits composés, et que le procédé consiste à mettre en contact lesdites fibres kératiniques sur lesquelles on a appliqué une composition tinctoriale contenant le ou lesdits colorants directs cationiques sélectionnés avec un gaz contenant de la vapeur d'eau dont la température est supérieure à 75°C, pendant un temps de contact entre ledit gaz et lesdites fibres à colorer inférieur à deux minutes.

A titre de naphtoquinone cationique utilisable selon l'invention, on peut par exemple citer, le chlorure de 2-bromo-4,8-diamino-6-(3'-triméthylammonium)-phénylamino-1,5-naphtoquinone référencé Basic Blue 99 (CI 56059).

Parmi les anthraquinones cationiques, on peut citer, à titre d'exemple, le chlorhydrate de 1-méthylamino-4-(γ-aminopropyl)-amino-anthraquinone, le méthylsulfate de 1 - ( N - méthyl - morpholinium - propylamino) - 4 - hydroxy - anthraquinone et le Basic Blue 22 (CI 61512).

Parmi les méthines cationiques, on peut citer à titre d'exemple, le Basic Yellow 29.

Parmi les colorants azoïques cationiques, on peut citer, à titre d'exemple, le chlorure de 1-(2'-méthoxyphénylazo)- 2 - hydroxy-7-triméthylammonium-naphtalène référencé Basic Red 76 (CI 12245), le chlorure de 4-(3'-triméthylammoniumphénylazo)-N-phényl-3-méthylpyrazolone-5 référencé basic Yellow 57 (CI 12719), le chlorure de 1-(4'-aminophénylazo)-2-hydroxy-7-triméthylammonium-naphtalène référencé Basic Brown 16 (CI 12250) et le 1-(3',2'-nitro-4'-aminophénylazo)-2-hydroxy-7-triméthylammonium-naphtalène référencé Basic Brown 17 (CI 12251).

Parmi les oxazines cationiques, on peut citer, à titre d'exemple, le Basic Blue 3 (CI 51004).

Les colorants directs cationiques utilisés selon le procédé de l'invention sont de préférence présents dans des concentrations allant de 0,01 à 10 % en poids et encore plus préférentiellement de 0,05 à 5 % en poids par rapport au poids total de la composition tinctoriale.

En plus de la vapeur d'eau, le gaz vecteur peut contenir de la vapeur de solvant, des gaz tels que l'oxygène, l'azote, des mélanges de gaz tels que l'air ou des composés vaporisables.

Les solvants utilisables pour la production de vapeur sont choisis parmi les solvants organiques cosmétiquement acceptables et plus particulièrement parmi les alcools tels que l'éthanol, l'isopropanol, l'alcool benzylique, l'alcool phényléthylique ou les glycols ou éthers de glycol tels que par exemple les éthers monométhylique, monoéthylique et monobutylique de l'éthylène glycol, le propylène glycol, le butylène glycol, le dipropylèneglycol ainsi que les alkyléthers comme le monobutyléther du diéthylèneglycol.

Le gaz comprend de préférence au moins 1% en volume de vapeur d'eau par rapport au volume total du gaz.

Le gaz est constitué de préférence soit uniquement ou essentiellement de vapeur d'eau, soit d'un mélange de vapeur d'eau et d'air.

La température du gaz est de préférence supérieure ou égale à 85°C et plus particulièrement comprise entre 85 et 150°C.

En particulier, le gaz est mis en contact avec la fibre à colorer pendant une durée allant de 0,01 seconde à 2 minutes.

De préférence, le gaz est mis en contact avec la fibre pendant une durée allant de 0,1 seconde à 50 secondes et encore plus préférentiellement de 1 à 10 secondes.
L'application du gaz peut être répétée plusieurs fois sur la même fibre, chaque opération se faisant selon la durée indiquée ci-dessus.

Dans un premier mode de mise en oeuvre du procédé selon l'invention qui est ici préféré, on applique sur les cheveux une composition tinctoriale contenant au moins un colorant direct cationique selon l'invention, puis on les soumet à l'action de la vapeur d'eau.

Selon d'autres modes de mise en oeuvre du procédé, il est également possible d'appliquer simultanément la composition tinctoriale et le gaz comprenant de la vapeur d'eau.

Il est également possible de faire parvenir sur les cheveux tout ou partie de la composition tinctoriale à l'aide du flux de gaz lorsque certains ou tous les constituants de la formule sont entraînables ou vaporisables.

L'application de vapeur d'eau est éventuellement suivie d'un rinçage à l'eau.

La production d'un gaz chaud comprenant de la vapeur d'eau peut se faire à l'aide de tout appareil connu en soi. Toutefois, selon la présente invention, on utilise de préférence un appareil tel que celui décrit dans la demande de brevet français FR-A-2273492, ou tout autre appareil équivalent, qui convient particulièrement bien.

La composition tinctoriale utilisée dans le procédé selon l'invention peut se présenter sous des formes habituellement utilisées pour la teinture des cheveux telles que de liquide plus ou moins épaissi ou gélifié, de crème, de mousse en aérosol ou sous toute autre forme appropriée pour réaliser une teinture des cheveux.

Les compositions tinctoriales utilisées conformément à l'invention sont généralement des compositions aqueuses pouvant contenir des ingrédients habituellement utilisés dans les compositions cosmétiques destinées à la coloration des cheveux, tels que des solvants, des agents tensioactifs, des épaississants, des agents traitants, des agents alcalinisants ou acidifiants, des agents conservateurs, des parfums ou tout autre additif utilisé dans ce type de composition.

La composition tinctoriale contenant au moins un colorant cationique présente un pH de préférence compris entre 2 et 11 et encore plus préférentiellement entre 5 et 9.

La composition tinctoriale peut aussi se présenter sous forme de solution anhydre ou de poudres qui sont diluées au moment de l'emploi avec de l'eau ou un support aqueux. Les poudres ainsi employées conduisent à un cataplasme. Les solutions anhydres peuvent être directement appliquées sur les cheveux humides. Ces supports sont par exemple décrits dans les demandes de brevet français FR-A-2500749 et FR-A-2598318, FR-A-2526031 et FR-A-2500748.

Les exemples qui suivent illustrent l'invention sans pour autant présenter un caractère limitatif.

### EXEMPLE 1(invention)

On prépare la composition tinctoriale suivante :

| | |
|---|---|
| - Mélange d'alcool cétylstéarylique et d'alcool cétylstéarylique oxyéthylèné à 33 moles d'oxyde d'éthylène (80/20) | 2 g |
| - Nonylphénol oxyéthyléné à 40 moles d'oxyde d'éthylène | 3 g |
| - Nonylphénol oxyéthyléné à 90 moles d'oxyde d'éthylène | 2 g |
| - Chlorure d'oléocétyl en C₁₆/C₁₈/C₁₈, 25/25/50) diméthylhydroxyéthylammonium en solution aqueuse à 30 % | 16,6 g |
| - 2-butoxyéthanol | 8 g |
| - Basic Blue 3 référencé Cl 51004 dans la 3ème édition du CTFA | 0,1 g |
| - Monoéthanolamine q.s. | pH 7 |
| - Eau déminéralisée q.s.p. | 100 g |

On applique cette composition tinctoriale sur une mèche de cheveux naturels c'est à dire non sensibilisés (mèche n° 1) et sur une mèche de ces mêmes cheveux ayant subi une permanente (mèche n° 2).

On envoie ensuite sur les deux mèches un jet de vapeur d'eau à 90 °C pendant 45 secondes. Les mèches sont ensuite rincées puis séchées.

Les nuances obtenues sont proches. La couleur des mèches est ensuite évaluée dans le système MUNSELL au moyen d'un colorimètre CM 2002 MINOLTA.
Selon la notation MUNSELL, une couleur est définie par l'expression H V / C dans laquelle les trois paramètres désignent respectivement la teinte (H), I'intensité (V) et la pureté (C), la barre oblique de cette expression est simplement une convention et n'indique pas un ratio.

La différence de couleur entre deux mèches est calculée en appliquant la formule de NICKERSON : Δ E = 0,4 CodH + 6dV + 3 dC, telle que décrite par exemple dans "Couleur, Industrie et Technique" pages 14-17 vol. n° 5 1978.

Dans cette formule, Δ E représente la différence de couleur entre deux mèches, dH, dV et dC représentent la variation en valeur absolue des paramètres H, V et C et Co représente la pureté de la mèche par rapport à laquelle on désire évaluer la différence de couleur.

Les résultats sont exprimés dans le tableau ci-après :

| **Couleur sur cheveux naturels** | **Couleur sur cheveux permanentés** | **Différence de couleur** | | | |
|---|---|---|---|---|---|
| | | dH | dV | dC | ΔE |
| 9,8 G 4,6 / 2,4 | 3,9 BG 4,6 / 3,2 | 4,1 | 0 | 0,8 | **6,3** |

L'écart de couleur entre la mèche de cheveux naturels (mèche n°1) et la mèche de cheveux permanentés (mèche n° 2) est faible, ce qui est significatif d'une coloration uniforme.

### EXEMPLE 2 (comparatif)

On applique la composition tinctoriale de l'exemple 1 sur une mèche de cheveux naturels non sensibilisés, identiques à ceux utilisés dans l'exemple 1 ci-dessus, (mèche n° 1) et sur une mèche de ces mêmes cheveux ayant subi une permanente (mèche n° 2).

Au lieu d'employer le procédé de l'invention, on laisse pauser la composition pendant 30 minutes à température ambiante. Les mèches sont ensuite rincées et séchées.

On évalue la différence de couleur entre les deux mèches comme à l'exemple 1. Les résultats sont exprimés dans le tableau ci-après :

| **Couleur sur cheveux naturels** | **Couleur sur cheveux permanentés** | **Différence de couleur** | | | |
|---|---|---|---|---|---|
| | | dH | dV | dC | ΔE |
| 1,9 BG 4,5 / 2,8 | 6,6 BG 4,6 / 4,8 | 4,7 | 0,1 | 2,0 | **11,9** |

On constate une différence de couleur importante entre les mèches de cheveux naturels et les mèches de cheveux permanentés, ce qui est significatif d'une coloration non uniforme et donc sélective suivant l'état de dégradation du cheveu.

### EXEMPLE 3 (invention)

On prépare la composition tinctoriale suivante :

| | |
|---|---|
| - Mélange d'alcool cétylstéarylique et d'alcool cétylstéarylique oxyéthylèné à 33 moles d'oxyde d'éthylène (80/20) | 2 g |
| - Nonylphénol oxyéthyléné à 40 moles d'oxyde d'éthylène | 3 g |
| - Nonylphénol oxyéthyléné à 90 moles d'oxyde d'éthylène | 2 g |
| - Chlorure d'oléocétyl en C₁₆/C₁₈/C₁₈, (25/25/50) diméthylhydroxyéthylammonium en solution aqueuse à 30 % | 16,6 g |
| - 2-butoxyéthanol | 8 g |
| - Basic Blue 99 référencé Cl 56059 dans la 3ème édition du CTFA | 0,15 g |
| - Monoéthanolamine q.s. | pH 7 |
| - Eau déminéralisée q.s.p. | 100 g |

On applique cette composition tinctoriale sur une mèche de cheveux naturels c'est à dire non sensibilisés (mèche n° 1) et sur une mèche de ces mêmes cheveux ayant subi une permanente (mèche n° 2).

On envoie ensuite sur les deux mèches un jet de vapeur d'eau à 90 °C pendant 45 secondes. les mèches sont ensuite rincées puis séchées.
On évalue ensuite la différence de couleur entre les deux mèches comme à l'exemple 1.

Les résultats sont exprimés dans le tableau ci-après :

| **Couleur sur cheveux naturels** | **Couleur sur cheveux permanentés** | **Différence de couleur** | | | |
|---|---|---|---|---|---|
| | | dH | dV | dC | ΔE |
| 2,2 BG 4,2 / 1,1 | 6,9 BG 4,3 / 1,3 | 4,7 | 0,1 | 0,2 | **3,3** |

L'écart de couleur entre la mèche de cheveux naturels (mèche n°1) et la mèche de cheveux permanentés (mèche n° 2) est faible, ce qui est significatif d'une coloration uniforme.

### EXEMPLE 4 (comparatif)

On applique la composition tinctoriale de l'exemple 3 sur une mèche de cheveux naturels non sensibilisés, identiques à ceux utilisés dans l'exemple 3 ci-dessus, (mèche n° 1) et sur une mèche de ces mêmes cheveux ayant subi une permanente (mèche n° 2).

Au lieu d'employer le procédé de l'invention, on laisse pauser la composition pendant 30 minutes à température ambiante. Les mèches sont ensuite rincées et séchées.

On évalue la différence de couleur entre les deux mèches comme à l'exemple 1. Les résultats sont exprimés dans le tableau ci-après :

| **Couleur sur cheveux naturels** | **Couleur sur cheveux permanentés** | **Différence de couleur** | | | |
|---|---|---|---|---|---|
| | | dH | dV | dC | ΔE |
| 8,6 BG 4,1 / 1,3 | 4,5 B 3,7 / 1,9 | 5,9 | 0,4 | 0,6 | **7,3** |

On constate que la différence de couleur entre les deux mèches est plus importante que celle obtenue à l'exemple 3 ci-dessus. La teinture obtenue selon ce procédé ne faisant pas partie de l'invention est donc moins uniforme que celle obtenue ci-dessus à l'exemple 3.

## Revendications

1. Procédé de teinture directe des fibres kératiniques mettant en oeuvre au moins un colorant direct cationique et un gaz contenant de la vapeur d'eau, caractérisé par le fait que ledit colorant direct cationique est choisi parmi les naphtoquinones et anthraquinones cationiques, les méthines cationiques, les azoïques cationiques, les oxazines cationiques et les sels desdits composés, et que le procédé consiste à mettre en contact lesdites fibres kératiniques sur lesquelles on a appliqué une composition tinctoriale contenant le ou lesdits colorants directs cationiques sélectionnés avec un gaz contenant de la vapeur d'eau dont la température est supérieure à 75°C, pendant un temps de contact entre ledit gaz et lesdites fibres à colorer inférieur à deux minutes.

2. Procédé selon la revendication 1, caractérisé par le fait que le colorant direct cationique est choisi parmi les méthines cationiques, les azoïques cationiques et les sels desdits composés.

3. Procédé selon les revendications 1 et 2, caractérisé par le fait que le gaz a une température supérieure ou égale à 85°C.

4. Procédé selon la revendication 3, caractérisé par le fait que le gaz a une température comprise entre 85 et 150°C.

5. Procédé selon l'une quelconque des revendications précédentes, caractérisé par le fait que le gaz est mis en contact avec la fibre à colorer pendant une durée allant de 0,01 seconde à 2 minutes.

6. Procédé selon la revendication 5, caractérisé par le fait que le gaz est mis en contact avec la fibre à colorer pendant une durée allant de 0,1 seconde à 50 secondes.

7. Procédé selon la revendication 6, caractérisé par le fait que le gaz est mis en contact avec la fibre à colorer pendant une durée allant de 1 seconde à 10 secondes.

8. Procédé selon l'une quelconque des revendications précédentes, caractérisé par le fait que l'application du gaz est répétée plusieurs fois sur une même fibre.

9. Procédé selon l'une quelconque des revendications précédentes, caractérisé par le fait que le gaz contient uniquement de la vapeur d'eau.

10. Procédé selon l'une quelconque des revendications 1 à 8, caractérisé par le fait que le gaz contient de la vapeur d'eau et au moins un autre composé sous forme de gaz ou de vapeur.

11. Procédé selon la revendication 10, caractérisé par le fait que le gaz contient de la vapeur d'eau et de l'air.

12. Procédé selon l'une quelconque des revendications précédentes, caractérisé par le fait que les colorants directs cationiques sont présents dans des concentrations allant de 0,01 à 10% en poids par rapport au poids total de la composition tinctoriale.

13. Procédé selon la revendication 12 caractérisé par le fait que les colorants directs cationiques sont présents dans des concentrations allant de 0,05 à 5% en poids par rapport au poids total de la composition tinctoriale.

## Claims

1. Process for the direct dyeing of keratin fibres, using at least one cationic direct dye and a gas containing water vapour, characterized in that the said cationic direct dye is chosen from cationic naphthoquinones and anthraquinones, cationic methines, cationic azo dyes, cationic oxazines and the salts of the said compounds, and in that the process consists in placing the said keratin fibres, on which a dye composition containing the said cationic direct dye(s) chosen has been applied, in contact with a gas containing water vapour whose temperature is above 75°C, for a contact time between the said gas and the said fibres to be dyed of less than two minutes.

2. Process according to Claim 1, characterized in that the cationic direct dye is chosen from cationic methines, cationic azo dyes and the salts of the said compounds.

3. Process according to Claims 1 and 2, characterized in that the temperature of the gas is greater than or equal to 85°C.

4. Process according to Claim 3, characterized in that the temperature of the gas is between 85 and 150°C.

5. Process according to any one of the preceding claims, characterized in that the gas is placed in contact with the fibre to be dyed for a period ranging from 0.01 second to 2 minutes.

6. Process according to Claim 5, characterized in that the gas is placed in contact with the fibre to be dyed for a period ranging from 0.1 second to 50 seconds.

7. Process according to Claim 6, characterized in that the gas is placed in contact with the fibre to be dyed for a period ranging from 1 second to 10 seconds.

8. Process according to any one of the preceding claims, characterized in that application of the gas is repeated several times on the same fibre.

9. Process according to any one of the preceding claims, characterized in that the gas contains water vapour exclusively.

10. Process according to any one of Claims 1 to 8, characterized in that the gas contains water vapour and at least one other compound in the form of gas or vapour.

11. Process according to Claim 10, characterized in that the gas contains water vapour and air.

12. Process according to any one of the preceding claims, characterized in that the cationic direct dyes are present in concentrations ranging from 0.01 to 10 % by weight relative to the total weight of the dye composition.

13. Process according to Claim 12, characterized in that the cationic direct dyes are present in concentrations ranging from 0.05 to 5 % by weight relative to the total weight of the dye composition.

## Patentansprüche

1. Verfahren zum direkten Färben von Keratinfasern unter Verwendung mindestens eines kationischen Direktfarbstoffes und eines Gases, das Wasserdampf enthält,
**dadurch gekennzeichnet, daß**
der kationische Direktfarbstoff unter den kationischen Naphthochinonen und Anthrachinonen, kationischen Methinen, kationischen Azofarbstoffen, kationischen Oxazinen und den Salzen dieser Verbindungen ausgewählt ist, und das Verfahren darin besteht, die Keratinfasern, auf die eine Zusammensetzung, die den oder die ausgewählten kationischen Direktfarbstoff(e) enthält, aufgetragen wurde, mit einem Gas, das Wasserdampf enthält, in Kontakt zu bringen, wobei die Temperatur des Gases über 75 °C liegt und die Kontaktzeit zwischen dem Gas und den zu färbenden Fasern weniger als 2 min beträgt.

2. Verfahren nach Anspruch 1, dadurch gekennzeichnet, daß der kationische Direktfarbstoff unter den kationischen Methinen, kationischen Azofarbstoffen und den Salzen dieser Verbindungen ausgewählt ist.

3. Verfahren nach Anspruch 1 und 2, dadurch gekennzeichnet, daß das Gas eine Temperatur von mindestens 85 °C aufweist.

4. Verfahren nach Anspruch 3, dadurch gekennzeichnet, daß das Gas eine Temperatur im Bereich von 85 bis 150 °C aufweist.

5. Verfahren nach einem der vorhergehenden Ansprüche, dadurch gekennzeichnet, daß das Gas mit der zu färbenden Faser während einer Zeitdauer von 0,01 s bis 2 min in Kontakt gebracht wird.

6. Verfahren nach Anspruch 5, dadurch gekennzeichnet, daß das Gas mit der zu färbenden Faser während einer Zeitdauer von 0,1 bis 50 s in Kontakt gebracht wird.

7. Verfahren nach Anspruch 6, dadurch gekennzeichnet, daß das Gas mit der zu färbenden Faser während einer Zeitdauer von 1 bis 10 s in Kontakt gebracht wird.

8. Verfahren nach einem der vorhergehenden Ansprüche, dadurch gekennzeichnet, daß die Anwendung des Gases mehrmals an der gleichen Faser wiederholt wird.

9. Verfahren nach einem der vorhergehenden Ansprüche, dadurch gekennzeichnet, daß das Gas nur Wasserdampf enthält.

10. Verfahren nach einem der Ansprüche 1 bis 8, dadurch gekennzeichnet, daß das Gas Wasserdampf und mindestens eine weitere Verbindung in Form von Gas oder Dampf enthält.

11. Verfahren nach Anspruch 10, dadurch gekennzeichnet, daß das Gas Wasserdampf und Luft enthält.

12. Verfahren nach einem der vorhergehenden Ansprüche, dadurch gekennzeichnet, daß die kationischen Direktfarbstoffe in Konzentrationen von 0,01 bis 10 Gew.-%, bezogen auf das Gesamtgewicht der Färbemittelzusammensetzung, vorliegen.

13. Verfahren nach Anspruch 12, dadurch gekennzeichnet, daß die kationischen Direktfarbstoffe in Konzentrationen von 0,05 bis 5 Gew.-%, bezogen auf das Gesamtgewicht der Färbemittelzusammensetzung, vorliegen.
